# EUROPÄISCHE PATENTSCHRIFT

(11) **EP 0 505 854 B1**
(45) Veröffentlichungstag und Bekanntmachung des Hinweises auf die Patenterteilung: **01.03.1995**
(21) Anmeldenummer: 92104374.1
(22) Anmeldetag: 13.03.1992
(51) Int. Cl.: A61K 7/06, A61K 7/09

(54) **Verfahren zur dauerhaften Verformung von Haaren**
Process for permanent waving of hair
Procédé pour l'ondulation permanente des cheveux

(30) Priorität: 26.03.1991 DE 4109869
(43) Veröffentlichungstag der Anmeldung: 30.09.1992
(73) Patentinhaber: Th. Goldschmidt AG, 45127 Essen (DE)
(72) Erfinder: Grüning, Burghard, Dr., W-4300 Essen 1 (DE); Leidreiter, Holger, Dr., W-4300 Essen 11 (DE)

(56) Entgegenhaltungen:
- EP-A- 0 295 780
- EP-A- 0 313 867
- EP-A- 0 346 151
- EP-A- 0 472 858
- DE-A- 1 492 174

## Beschreibung

Die Erfindung betrifft ein Verfahren zur dauerhaften Verformung von Haaren durch Einwirken von keratinreduzierenden Verformungsmitteln, gegebenenfalls Spülen mit Wasser und nachfolgende Fixierung mit oxidierenden Mitteln.

Der erste Verfahrensschritt beim Dauerwellprozeß ist die teilweise Reduktion des Cystins des Haares zum Cystein. Neben den Disulfidbrücken sollen auch salzartige Bindungen und Wasserstoffbrücken gelockert werden. Die reduktiven Dauerwellmittel liegen daher zumeist in wäßriger, alkalischer Lösung vor. Als Reduktionsmittel finden vorzugsweise Salze der Thioglykolsaure Verwendung. Weniger häufig werden als reduktive Wirkstoffe Cystein, Cysteamin, Sulfid, Thioglykolsäuremonoglycerinester, Thiomilchsäure oder Thioglycerin verwendet.

In einem nachfolgenden oxidativen Verfahrensschritt werden die im ersten Schritt entstandenen SH-Gruppen zu Disulfidbrücken rekombiniert, wodurch die Verformung fixiert wird.

Das wichtigste Oxidationsmittel in diesem Prozeß ist Wasserstoffperoxid, daneben kommen auch andere oxidative Fixiermittel, wie Bromate, Percarbamide oder Natriumperborat, zum Einsatz. Dauerwell- und Fixiermittel werden unter Zusatz zahlreicher Hilfsstoffe, wie Farbstoffen, Duftstoffen, Färbungsmitteln, Netzmitteln, Emulgatoren, Carriern und weiteren Stoffen, die der Haarpflege dienen, wie Konditioniermitteln, Ölen, Kräuterextrakten oder Eiweißhydrolysaten formuliert.

Allen Verfahren zur dauerhaften Verformung der Haare ist gemeinsam, daß der Prozeß eine wesentliche Belastung für Haar und Haut darstellt und daß Versuche zur Reduzierung der Haar- und Hautschädigung mit einer Verschlechterung des Wellergebnisses verbunden sind. Es bestehen daher schon lange Bemühungen, einen Dauerwellprozeß zu entwickeln, der das Wellergebnis verbessert und zugleich das Haar weniger schädigt.

In der Vergangenheit sind zahlreiche Vorschläge zur Verbesserung des Dauerwellprozesses gemacht worden, die sich einerseits auf die Verbesserung des Wellergebnisses und andererseits auf die Verbesserung der physiologischen Verträglichkeit der Präparate und des Prozesses beziehen, wobei natürlich auch ein besseres Wellergebnis indirekt zur Verbesserung der Verträglichkeit einfach dadurch beitragen kann, daß eine Dauerwellbehandlung erst in zeitlich größeren Abständen notwendig wird. Eine Übersicht über Dauerwellverfahren kann W. Umbach, Kosmetik, Georg Thieme Verlag Stuttgart, 1988, entnommen werden.

In der DE-OS 37 13 559 wird die Verwendung einer Siliciumdioxid/Dimethylpolysiloxan-Mischung in Mitteln zur dauerhaften Verformung oder zur Entfernung von Haaren beschrieben. Die Siliciumdioxid/Dimethylpolysiloxan-Mischung kann sowohl der reduzierenden Dauerwellösung als auch dem oxidativen Fixiermittel zugesetzt werden. Durch die Anwendung der Mittel wird ein gleichmäßig verformtes, glänzendes Haar mit hoher Sprungkraft und Elastizität erhalten. Diese Mittel weisen jedoch verschiedene Nachteile auf: Die Siliciumdioxid/Dimethylpolysiloxan-Mischungen müssen in den wäßrigen Zubereitungen emulgiert werden. Diese Emulsionen sind jedoch häufig nicht ausreichend stabil, so daß sie oft kurz vor Anwendung hergestellt werden müssen. Nur unter diesen Voraussetzungen ist eine gleichmäßige Verteilung der Siliciumdioxid/Dimethylpolysiloxan-Mischung auf dem Haar gewährleistet, so daß oft Gefahr besteht, daß das Behandlungsergebnis ungleichmäßig ausfällt.

In den deutschen Patentschriften 33 23 881 und 37 35 086 werden Organopolysiloxane mit Buntesalzgruppen beschrieben, die zur Verwendung der Oberflächenbehandlung von anorganischen oder organischen Materialien dienen können. Weiter ist aus der DE-PS 37 35 086 bekannt, solche Organopolysiloxane mit Buntesalzgruppen auch bei der Behandlung tierischer und menschlicher Haare einzusetzen.

Dabei werden Haare mit Lösungen von Silicon-Buntesalzen, die 10 % Wirkstoff und 8 % Thioglykolsäure enthalten, behandelt. Diese Lösungen weisen einen sauren pH-Wert auf. Durch die Anwendung der Silicon-Buntesalze, insbesondere in Kombination mit Thioglykolsäure, wird der Griff der Haare verbessert. Eine dauerhafte Verformung der Haare wird in dieser Patentschrift nicht erwähnt. Sie wäre auch mit derartigen sauren Lösungen nicht zu erreichen.

Es ist bekannt, daß die Qualität des Wellergebnisses durch die Anwendung alkalischer Lösungen wesentlich verbessert wird. Verändert man den pH-Wert der Silicon-Buntesalze/Thioglykolsäure-Lösungen durch Zugabe von Ammoniak auf pH 7 oder gar pH 9, so scheidet sich bereits nach wenigen Minuten bis Sekunden aus ihnen ein vernetztes, unlösliches Polymer ab. Bei Anwendung dieser unbeständigen Lösungen auf dem Haar werden zahlreiche weiße, schwer entfernbare Partikel gebildet.

Die vorliegende Erfindung befaßt sich mit dem technischen Problem, ein Verfahren zur dauerhaften Verformung der Haare zu finden, welches das Wellergebnis verbessert und die vorgenannten Nachteile nicht aufweist und welches insbesondere die Verwendung von Mitteln beinhaltet, die aus einer wäßrigen Lösung oder stabilen Dispersion angewendet werden können.

Erfindungsgemäß gelingt dies durch ein Verfahren, welches dadurch gekennzeichnet ist, daß man vor oder während der Behandlung mit dem oxidierenden Mittel auf die Haare eine flüssige Zubereitung mit einem Gehalt von 0,1 bis 25 Gew.-% Silicon-Buntesalzen einwirken läßt.

Dabei kann zweckmäßigerweise die flüssige Zubereitung mit ihrem wirksamen Gehalt an Silicon-Buntesalzen zusammen mit den für die Fixierung notwendigen oxidierenden Behandlungsmitteln zugesetzt werden. Als oxidierende Behandlungsmittel können dabei in üblicher Weise wäßrige Hydroxyperoxid-Lösungen oder Lösungen von Alkalibromaten, wie z. B. Kalium- und Natriumbromat eingesetzt werden. Diese Mittel können Zusätze, wie Säuren oder Peroxidstabilisatoren, enthalten.

Dabei wird die Silicon-Buntesalzzubereitung bevorzugt in einer wäßrigen Lösung eingesetzt. Es können aber auch alkoholische oder wäßrig/alkoholische Lösungen verwendet werden. Vorzugsweise wird dabei die flüssige Zubereitung in Form einer wäßrigen Lösung mit einem Gehalt von 0,5 bis 15 Gew.-% an Silicon-Buntesalzen eingesetzt.

In einer anderen Ausführungsform des erfindungsgemäßen Verfahrens wird die flüssige Zubereitung mit dem Silicon-Buntesalz in einem zusätzlichen Behandlungsschritt vor der Fixierung mit den oxidierenden Mitteln auf das Haar aufgebracht. Die Zubereitung kann ferner die in der Haarkosmetik üblichen Zusätze, wie Tenside, Netzmittel, pflegende Zusätze, Öle, Wachse, Duftstoffe und Konservierungsmittel, enthalten.

Die Silicon-Buntesalze sind Organopolysiloxane mit Buntesalzgruppen, die durch folgende allgemeine Formel wiedergegeben werden können 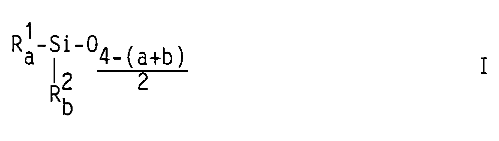
wobei
- R¹: der Methylrest ist, jedoch bis zu 10 % der Gruppen R¹ Phenylreste sein können,
- R²: der Rest R³
- R³ =: 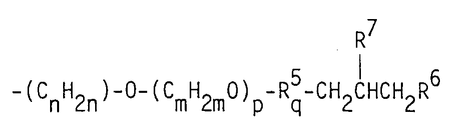
- R⁵ =: 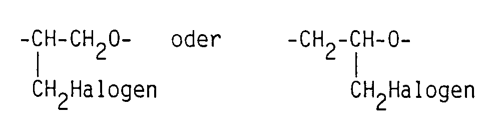
- R⁶, R⁷ =: eine der beiden Gruppen -OH, die andere -S₂O₃Me
- Me =: Alkalimetall oder gegebenenfalls substituierter Ammoniumrest,
- n =: 3 bis 6,
- m =: 2 bis 3,
- p =: 0 bis 100,
- q =: 0 bis 5, und/oder
der Rest R⁴
- R⁴ =: -(CₓH₂ₓ)-O-(C_{y}H_{2y}O)_{z}R⁸
- R⁸ =: Wasserstoff-, Alkyl- oder Acylrest mit 1 bis 4 Kohlenstoffatomen,
- x =: 3 bis 6,
- y =: 2 bis 3,
- z =: 1 bis 100, ist,
mit der Maßgabe, daß im durchschnittlichen Molekül mindestens 1 Rest R² die Bedeutung des Restes R³ hat,
- a =: 1 bis 2,33,
- b =: 0,02 bis 1 und
2 ≦ a + b ≦ 3 ist.

Von besonderer Bedeutung ist in dieser Formel die Definition des Restes R². Dieser Rest R² kann Zwei verschiedene Reste, nämlich den Rest R³ oder den Rest R⁴ bedeuten.

Bevorzugt sind Organopolysiloxane, bei denen der Rest R² die Bedeutung des Restes R³ hat.

Innerhalb des Restes R³ stellt die Gruppe CₙH₂ₙ einen Alkylenrest dar, welcher 3 bis 6 Kohlenstoffatome aufweist. Dieser Alkylenrest kann geradkettig oder verzweigt sein. Vorzugsweise ist der Alkylenrest -(CH₂)₃-. Der Rest R³ ist somit über Kohlenstoff an das Siliciumatom gebunden. Die Gruppe CₘH₂ₘO ist eine Oxyalkylengruppe. Der Index m kann einen Zahlenwert von 2 oder 3 annehmen, je nachdem, ob dieser Rest die Bedeutung einer Oxyethylen- oder Oxypropylengruppe hat. Da die Formel I jedoch eine allgemeine durchschnittliche Formel ist und im mittleren Molekül sowohl Oxyethylen- wie Oxypropyleneinheiten nebeneinander vorliegen können, kann der Wert des Index m zahlenmäßig auch einen gebrochenen Wert zwischen 2 und 3 annehmen. Die Anzahl der Oxyalkyleneinheiten ist durch den Index p wiedergegeben. Der Index p hat im mittleren Molekül einen Wert von 0 bis 100.

R⁵ wird durch die Reste 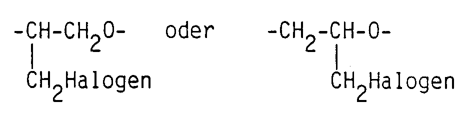
gebildet. Halogen steht hier für Halogenreste, vorzugsweise den Chlorrest. Die Anzahl der Reste R⁵ wird durch den Index q wiedergegeben. q kann einen Wert von 0 bis 5 haben. Die Reste R⁵ sind herstellungsbedingt und tragen zu den Eigenschaften der erfindungsgemäßen Organopolysiloxane nicht oder nicht wesentlich bei.

Einer der beiden Reste R⁶ bzw. R⁷ hat die Bedeutung einer Buntesalzgruppe -S₂O₃Me, wobei Me ein Alkalimetall, vorzugsweise Natrium, oder ein gegebenenfalls substituierter Ammoniumrest ist. Beispiele von substituierten Ammoniumresten sind der Tetrabutylammonium-, Tetramethylammonium- oder Didecyldimethylammoniumrest. Der andere Rest R⁶ bzw. R⁷ ist ein Hydroxylrest.

R² kann gegebenenfalls auch der Rest R⁴ sein. Dabei ist zu beachten, daß die Anwesenheit mindestens eines Restes R³ zwingend ist.

Innerhalb des Restes R⁴ entspricht die Gruppe CₓH₂ₓ der Gruppe CₙH₂ₙ des Restes R³. Beide Gruppen müssen jedoch nicht identisch sein. In ähnlicher Weise entspricht die Gruppe (C_{y}H_{2y}O)_{z} des Restes R⁴ der Gruppe (CₘH₂ₘO)ₚ des Restes R³, ohne daß diese beiden Gruppen identisch sein müssen. R⁸ ist ein Wasserstoff-, Alkyl- oder Acylrest mit 1 bis 4 Kohlenstoffatomen. Als Alkylrest ist der Methylrest und als Acylrest der Acetylrest bevorzugt.

Beispiele von erfindungsgemäßen modifizierten Organopolysiloxanen können durch die folgenden Formeln wiedergegeben werden, wobei es sich, dem Fachmann verständlich, um durchschnittliche, idealisierte Formeln handelt. Es liegt im Wesen der siliciumorganischen Chemie, daß derartige Organopolysiloxane immer in Form von Gemischen, z.B. unterschiedlicher Länge der Siloxanblöcke, vorliegen. 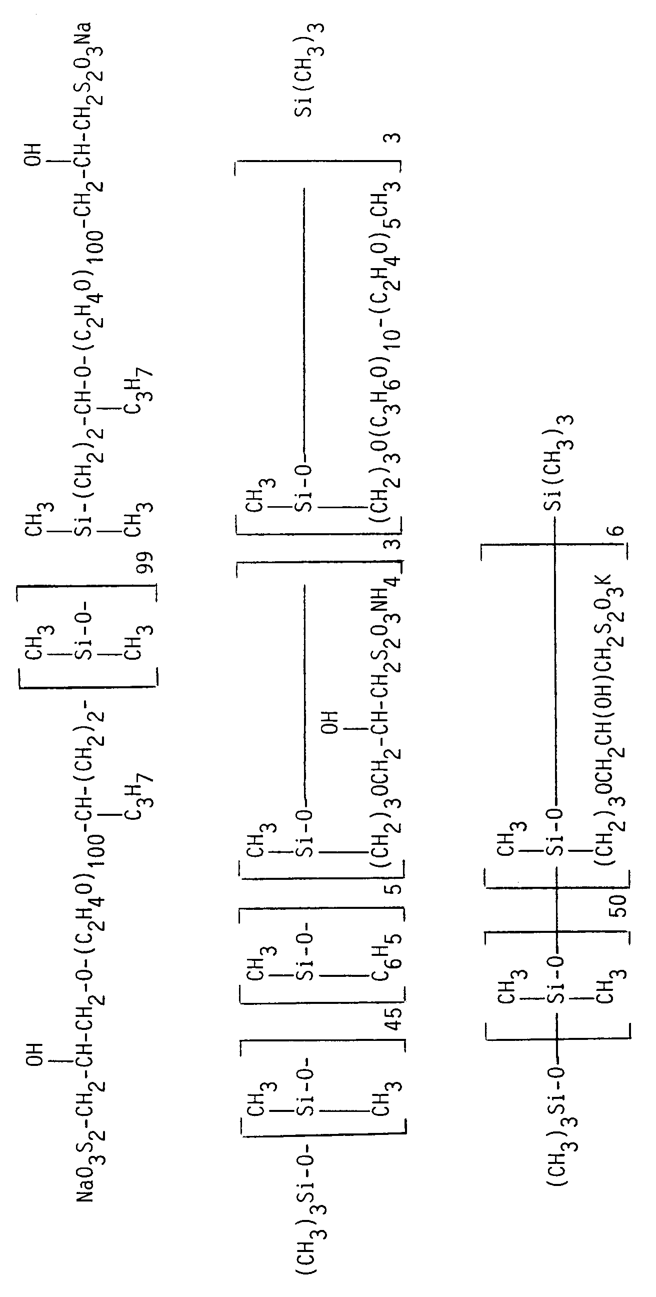
Besonders bevorzugt sind Verbindungen der allgemeinen Formel I, bei denen der Rest R² = R³ ist und der die Bedeutung 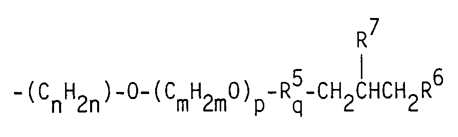
hat, und
dort die Indices p und q = 0 sind.

Das im ersten Schritt des Verfahrens angewendete keratinerweichende, reduzierende Mittel kann beliebig aus den im Stand der Technik angebotenen Mitteln ausgewählt werden. Besonders gute Resultate wurden mit solchen Mitteln erzielt, deren pH-Wert bei 6,5 bis 9,5 liegt, wobei ein pH-Wert von 7,5 bis 9,0 besonders bevorzugt ist.

Selbstverständlich kann das auf einem keratinerweichenden Wirkstoff basierende Mittel zur dauerhaften Haarverformung als auch das auf einem oxidierenden Wirkstoff basierende Mittel zur dauerhaften Haarverformung alle für ein derartiges Mittel üblichen und bekannten Zusatzstoffe, zum Beispiel Verdickungsmittel, wie beispielsweise Kaolin, Bentonit, Fettsäuren, höhere Fettalkohole, Stärke, Polyacrylsäure und deren Derivate, Cellulosederivate, Alginate, Vaseline oder Paraffinöl, Netzmittel oder Emulgatoren aus den Klassen der anionischen, kationischen, amphoteren oder nichtionogenen oberflächenaktiven Substanzen, beispielsweise Fettalkoholsulfate, Fettalkoholethersulfate, Alkylsulfonate, Alkylbenzolsulfate, quartäre Ammoniumsalze, Alkylbetaine, oxethylierte Fettalkohole, oxethylierte Alkylphenole, Fettsäurealkanolamide oder oxethylierte Fettsäureester, ferner Trübungsmittel, wie zum Beispiel Polyethylenglykolester, oder Alkohole, wie beispielsweise Ethanol, Propanol, Isopropanol oder Glycerin, Lösungsvermittler, Stabilisatoren, Puffersubstanzen, Parfümöle, Farbstoffe sowie haarkonditionierende und haarpflegende Bestandteile, wie zum Beispiel kationische Polymere, Lanolinderivate, Cholesterin, Pantothensäure, Betain oder Polydimethylsiloxane enthalten. Die erwähnten Bestandteile werden in den für solche Zwecke üblichen Mengen verwendet, zum Beispiel die Netzmittel und Emulgatoren in Konzentrationen von etwa 0,2 bis 10 Gew.-%, während die Verdickungsmittel in einer Menge von etwa 1,0 bis 25 Gew.-% in diesem Mittel enthalten sein können.

Weiterhin können diesem Mittel zusätzlich bekannte wirkungsverstärkende Stoffe, wie zum Beispiel Dipropylenglykolmonomethylether oder 2-Pyrrolidon, in einer Menge von etwa 2 bis 30 Gew.-% zugesetzt werden.

Durch den Einsatz der Silicon-Buntesalze enthaltenden Zubereitung vor oder während der oxidativen Nachbehandlung wird die Wirkung dieser Fixierungsmittel wesentlich gesteigert. So verbessert sich das Wellergebnis erheblich, wobei die Haarkrause verstärkt und die Verformung am Haaransatz, Mittelstück, Deckhaar und Haarspitze sichtlich stabilisiert werden. Zusätzlich wird eine leichtere Kämmbarkeit, eine Erhöhung der Geschmeidigkeit und des Glanzes der Haare erzielt.

Die Wirkungsweise der Silicon-Buntesalze bei der Haarbehandlung läßt sich durch folgende Reaktionsmöglichkeiten erklären:
Durch die zunächst reduktive Behandlung des Haares werden Cystein und dabei freie SH-Gruppen gebildet. Diese SH-Gruppen können mit den Thiosulfatgruppen der Silicon-Buntesalze gemäß 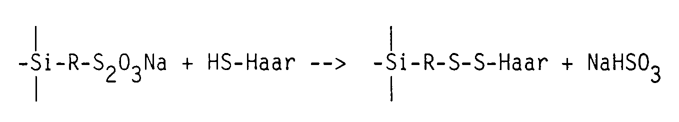
reagieren. Danach erfolgt eine Umlagerung zu den symmetrischen Disulfiden gemäß: 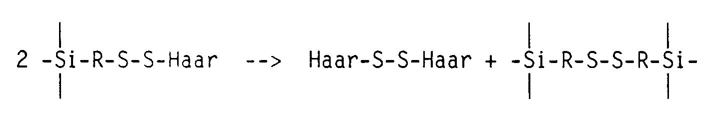
wodurch die Cystinbrücken des Haares zurückgebildet werden und sich zugleich ein unlöslicher, vernetzter Polymerfilm auf dem Haar bildet, der eine zugleich schützende und hydrophobierende Funktion ausübt.

Anhand der nachfolgenden Beispiele wird das erfindungsgemäße Verfahren noch näher erläutert.

### Beispiel 1

### Dauerwellanwendung mit Silicon-Buntesalz in der oxidierenden Fixierlösung

### Wellmittel:

| | |
|---|---|
| Thioglycolsäure (99 %ig) | 11,58 g |
| Ammoniumhydrogencarbonat | 4,2 g |
| Ammoniumhydroxid (25 %ig) | 6,4 g |
| Wasser | 77,82 g |
| | 1̅0̅0̅,̅0̅0̅ g̅ |

Der pH-Wert beträgt 8,5.

### Fixiermittel:

| | |
|---|---|
| Wasserstoffperoxid (30 %ig) | 7,33 g |
| Wasser | 82,67 g |
| und | 10,00 g einer |

50 %igen wäßrigen/alkoholischen Lösung eines Silicon-Buntesalzes der mittleren Formel: 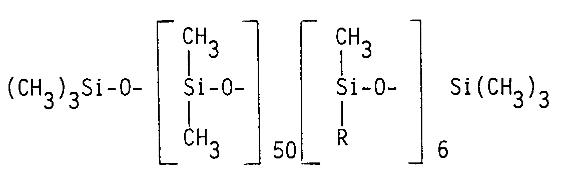
- R =: 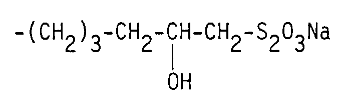
Der pH-Wert des Fixiermittels wird mit Zitronensäure auf 3,5 eingestellt.

Das Haar wird mit einem milden Shampoo gewaschen. Anschließend werden in das handtuchtrockene Haar Wickler eingedreht. Je nach Länge des Haares wird mit 50 bis 60 ml des keratinrezudierenden Wellmittels das Haar durchgefeuchtet. Nach 25 Minuten Einwirkzeit wird das gewickelte Haar mit Wasser gespült und trockengetupft. Daraufhin wird das Silicon-Buntesalz enthaltende Fixiermittel in einer Menge von etwa 50 bis 60 ml aufgetragen. Das Fixiermittel wird unmittelbar vor Anwendung aus den einzelnen Komponenten zusammengestellt. Nach einer Einwirkzeit von 10 Minuten werden die Wickler entfernt. Das Haar wird mit Wasser gespült und getrocknet.

Das so behandelte Haar besitzt eine gleichmäßige, elastische Krause, die eine gute, lang anhaltende Stabilität auch unter feuchtem Klima aufweist.

### Beispiel 2

### Dauerwellanwendung mit Silicon-Buntesalz in einem zusätzlichen Anwendungsschritt vor der Fixierbehandlung

- Wellmittel wie in Beispiel 1,
- Silicon-Buntesalzzubereitung wie in Beispiel 1,
- Fixiermittel:

| | |
|---|---|
| Wasserstoffperoxid (30 %ig) | 7,33 % |
| Wasser | 92,67 % |

Der pH-Wert des Fixiermittels wird mit Zitronensäure auf 3,5 eingestellt.

Das Haar wird mit einem milden Shampoo gewaschen. Anschließend werden in das handtuchtrockene Haar Wickler eingedreht. Je nach Länge des Haares wird mit 50 bis 60 ml des Wellmittels das Haar durchgefeuchtet. Nach 20 Minuten Einwirkzeit wird das gewickelte Haar mit Wasser gespült und trockengetupft. Danach wird das Haar mit 60 ml der Silicon-Buntesalzzubereitung durchgefeuchtet. Nach einer weiteren Einwirkdauer von 15 Minuten wird das gewickelte Haar erneut mit Wasser gespült und trockengetupft.

Anschließend wird das oxidierende Fixiermittel in einer Menge von 60 bis 80 ml aufgetragen. Nach einer Einwirkzeit von 10 Minuten werden die Wickler entfernt. Das Haar wird mit Wasser gespült und getrocknet.

Das so behandelte Haar besitzt eine gleichmäßige Krause mit guter Elastizität, deren Wellergebnis vor allem auch am Haaransatz gut ausgeprägt ist, und auch nach vier Wochen noch deutlich verbessert ist gegenüber dem Haar, welches mit einem Verfahren nach dem Stand der Technik behandelt wurde.

## Patentansprüche

1. Verfahren zur dauerhaften Verformung von Haaren durch Einwirken von keratinreduzierenden Verformungsmitteln, gegebenenfalls Spülen mit Wasser und nachfolgende Fixierung mit oxidierenden Mitteln, dadurch gekennzeichnet, daß man vor oder während der Fixierung mit den oxidierenden Mitteln auf die Haare eine flüssige Zubereitung mit einem Gehalt von 0,1 bis 25 Gew.-% Silicon-Buntesalzen einwirken läßt.

2. Verfahren nach Anspruch 1, dadurch gekennzeichnet, daß man eine wäßrige Zubereitung mit einem Gehalt von 0,5 bis 15 Gew.-% Silicon-Buntesalzen verwendet.

3. Verfahren nach Anspruch 1 oder 2, dadurch gekennzeichnet, daß man als Silicon-Buntesalze Verbindungen der allgemeinen durchschnittlichen Formel 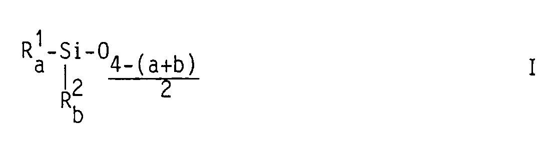 wobei
R¹ der Methylrest ist, jedoch bis zu 10 % der Gruppen R¹ Phenylreste sein können,
R² der Rest R³
R³ = 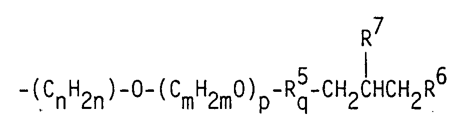
R⁵ = 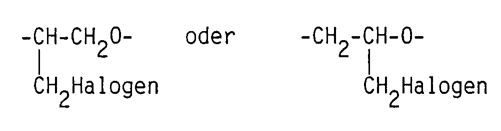
R⁶, R⁷ = eine der beiden Gruppen -OH, die andere -S₂O₃Me
Me = Alkalimetall oder gegebenenfalls substituierter Ammoniumrest,
n = 3 bis 6,
m = 2 bis 3,
p = 0 bis 100,
q = 0 bis 5, und/oder
der Rest R⁴
R⁴ = -(CₓH₂ₓ)-O-(C_{y}H_{2y}O)_{z}R⁸
R⁸ = Wasserstoff-, Alkyl- oder Acylrest mit 1 bis 4 Kohlenstoffatomen,
x = 3 bis 6,
y = 2 bis 3,
z = 1 bis 100, ist,
mit der Maßgabe, daß im durchschnittlichen Molekül mindestens 1 Rest R² die Bedeutung des Restes R³ hat,
a = 1 bis 2,33,
b = 0,02 bis 1 und
2 ≦ a + b ≦ 3 ist, verwendet.

4. Verfahren nach Anspruch 3, dadurch gekennzeichnet, daß man als Silicon-Buntesalze Verbindungen verwendet, in denen in der allgemeinen Formel I der Rest R² = R³ ist und dort die Indices p und q = 0 sind.

## Claims

1. Process for the permanent setting of hair by the action of keratin-reducing setting compositions, optional rinsing with water and subsequent fixing with oxidizing agents, characterized in that, before or during the fixing with the oxidizing agents, a livid preparation having a content of 0.1 to 25 % by weight of silicone Bunte salts is allowed to act on the hair.

2. Process according to Claim 1, characterized in that an aqueous preparation containing from 0.5 to 15 % by weight of silicone Bunte salts is used.

3. Process according to Claim 1 or 2, characterized in that the silicone Bunte salts used are compounds of the general average formula 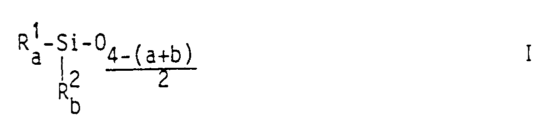 where
R¹ is the methyl radical, but up to 10 % of the groups R¹ can be phenyl radicals,
R² is the radical R³
R³ = 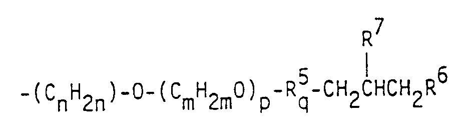
R⁵ = 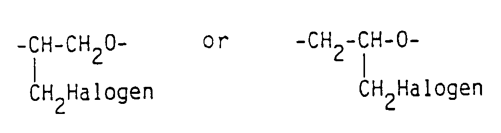
R⁶ and R⁷ = one of the two groups -OH, the other -S₂O₃Me
Me = alkali metal or optionally substituted ammonium radical,
n = 3 to 6,
m = 2 to 3,
p = 0 to 100,
q = 0 to 5, and/or
the radical R⁴
R⁴ = -(CₓH₂ₓ)-O-(C_{y}H_{2y}O)_{z}R⁸
R⁸ = hydrogen, alkyl, or acyl radical having 1 to 4 carbon atoms,
x = 3 to 6,
y = 2 to 3,
z = 1 to 100,
with the proviso that in the average molecule at least 1 radical R² has the meaning of the radical R³,
a = 1 to 2.33,
b = 0.02 to 1 and
2 ≦ a + b ≦ 3.

4. Process according to Claim 3, characterized in that the silicone Bunte salts used are compounds in which in the general formula I the radical R² = R³ and the indices p and q = 0 there.

## Revendications

1. Procédé pour l'ondulation permanente des cheveux grâce à l'action d'agents de mise en forme réduisant la kératine, éventuellement un rinçage avec de l'eau et une fixation subséquente avec des agents oxydants, caractérisé en ce qu'on laisse agir sur les cheveux une préparation liquide contenant entre 0,1 et 25 % en poids de silicium-sels de Bunte, avant ou pendant la fixation avec les agents oxydants.

2. Procédé selon la revendication 1, caractérisé en ce qu'on utilise une préparation aqueuse contenant entre 0,5 et 15 % en poids de silicium-sels de Bunte.

3. Procédé selon la revendication 1 ou 2, caractérisé en ce qu'on utilise, comme composés de silicium-sels de Bunte, des composés répondant à la formule générale moyenne : 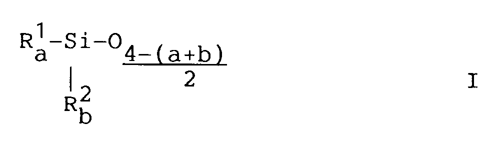 dans laquelle :
R¹ est le reste méthyle, mais jusqu'à 10 % des groupes R¹ peuvent être des restes phényle,
R² est le reste R³
R³ = 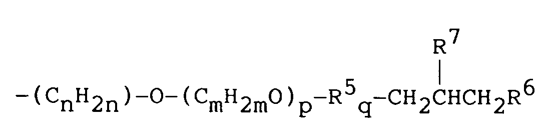
R⁵ = 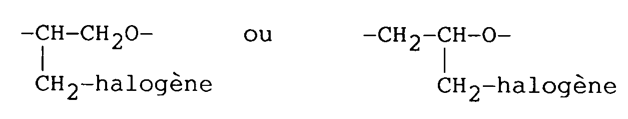
R⁶, R⁷ = un des deux groupes signifie -OH, et l'autre groupe signifie -S₂O₃Me,
Me = un métal alcalin ou un reste d'ammonium éventuellement substitué,
n = 3 à 6,
m = 2 à 3,
p = 0 à 100,
q = 0 à 5,
et/ou
R² est le reste R⁴
R⁴ = -(CₓH₂ₓ)-O-(C_{y}H_{2y}O)_{z}R⁸
R⁸ = un reste d'hydrogène, un reste alkyle ou acyle ayant de 1 à 4 atomes de carbone,
x = 3 à 6,
y = 2 à 3,
z = 1 à 100,
avec la condition qu'au moins 1 reste R² ait la signification du reste R³ dans la molécule moyenne,
a = 1 à 2,33,
b = 0,02 à 1, et
2 ≦ a + b ≦ 3.

4. Procédé selon la revendication 3, caractérisé en ce qu'on utilise, comme composés de silicium-sels de Bunte, des composés dans lesquels, dans la formule générale I, le reste R² = R³, et où les indices p et q = 0.
